# FASCICULE DE BREVET EUROPEEN

(11) **EP 2 948 079 B1**
(45) Date de publication et mention de la délivrance du brevet: **25.01.2017**
(21) Numéro de dépôt: 13808086.6
(22) Date de dépôt: 20.11.2013
(51) Int. Cl.: A61B 17/72, A61B 17/70, A61F 2/30

(54) **IMPLANT A ALLONGEMENT AUTOMATIQUE**
SELBSTEXPANDIERENDES IMPLANTAT
SELF-EXTENDING IMPLANT

(30) Priorité: 23.01.2013 FR 1350585
(43) Date de publication de la demande: 02.12.2015
(73) Titulaire: Euros, 13600 La Ciotat (FR)
(72) Inventeur: MILADI, Lotfi, 92340 Bourg La Reine (FR)
(74) Mandataire: Orsini, Fabienne
(86) Numéro de dépôt international: PCT/FR2013/052798
(87) Numéro de publication internationale: WO 2014/114853

(56) Documents cités:
- WO-A1-2012/085405
- CA-A1- 2 802 078
- US-A- 5 516 335
- US-A1- 2010 063 545

## Description

### DOMAINE TECHNIQUE AUQUEL SE RAPPORTE L'INVENTION

La présente invention concerne de manière générale un implant à fixer à un os d'un jeune patient.

Elle concerne plus particulièrement un implant à allongement automatique, comportant :
- un corps délimitant intérieurement un logement axial qui s'ouvre d'un côté par une embouchure et qui présente du côté opposé un rétrécissement de section formant sur le corps une face de butée,
- un bouchon annulaire qui est fixé à l'embouchure du logement axial dudit corps et qui présente une face d'arrêt tournée vers ladite face de butée,
- au moins une tige engagée au travers dudit bouchon annulaire et du logement axial dudit corps, et
- une bague fendue qui est montée sur la tige et qui est logée dans le logement axial dudit corps.

L'invention trouve une application particulièrement avantageuse dans la réalisation d'un implant de connexion de deux vertèbres, d'un implant de connexion de deux parties d'un os long, ou encore d'un implant équipé d'une prothèse articulaire.

### ARRIERE-PLAN TECHNOLOGIQUE

Les opérations pratiquées sur les os des jeunes patients présentent souvent un même inconvénient, qui est de limiter ou de bloquer ensuite la croissance de ces os.

Par exemple, dans le cas d'une résection d'une tumeur au genou, il est connu de remplacer une partie de l'os et de son articulation par une prothèse. Malheureusement, c'est la partie de l'os située près de l'articulation du genou qui présente le plus fort potentiel de croissance. Dès lors, la jambe opérée du patient s'allongera moins que l'autre, ce qui provoquera un déséquilibre.

La solution actuellement mise en oeuvre pour remédier à ce problème consiste alors à réopérer régulièrement le patient de manière à allonger sa prothèse. On comprend que cette solution n'est pas satisfaisante car ces opérations répétées sont autant de traumatismes pour le patient, en plus des risques qu'elles lui engendrent.

Par ailleurs, dans le cas d'une déviation importante de la colonne vertébrale (scoliose ou cyphose), il est connu d'utiliser des tiges de liaison rigides permettant de redresser la colonne vertébrale. Pour ce faire, les tiges de liaison sont cintrées suivant une courbure adéquate, puis sont bloquées parallèlement à la colonne vertébrale au moyen d'une pluralité de crochets et de vis fixés à certaines des vertèbres de la colonne vertébrale. Le risque est alors que ces tiges de liaison empêchent toute croissance de la colonne vertébrale, nécessitant des interventions répétées tous les six mois pour allonger ces tiges et permettre la poursuite de la croissance de la colonne vertébrale de l'enfant. Ces interventions itératives comportent des risques de complications fréquentes comme la fusion des vertèbres, bloquant ainsi toute mobilité de la colonne vertébrale.

Une solution connue pour accompagner l'allongement de la colonne vertébrale sans nouvelle intervention est présentée dans le document US2009/0204156.

Elle consiste à connecter deux tiges parallèles par un mécanisme qui laisse les deux tiges libres de se translater lorsqu'elles sont tirées à l'opposé l'une de l'autre mais qui bloque ces deux tiges lorsqu'elles sont poussées l'une vers l'autre. Ainsi, l'implant est-il libre de s'allonger lorsque le patient grandit, mais il ne peut se raccourcir, si bien qu'il assure sa fonction de redressement de la colonne vertébrale.

Le mécanisme décrit dans ce document comporte un corps traversé par deux logements parallèles qui accueillent chacun l'une des deux tiges. Chaque logement s'ouvre d'un côté du corps par une large embouchure, et présente une section qui se réduit progressivement jusqu'à l'autre côté du corps.

Une bague fendue est alors montée sur chaque tige et est maintenue à l'intérieur de chaque logement du corps par un bouchon. Chaque bouchon est plus précisément découpé pour former un ressort qui comprime chaque bague fendue en appui contre le rétrécissement de section de chaque logement du corps.

Ainsi, lorsque les tiges sont poussées l'une vers l'autre, elles poussent les bagues fendues dans les rétrécissements de section des logements du corps, si bien que ces bagues fendues se compriment sur les tiges et bloquent ces dernières.

Au contraire, lorsque les tiges sont tirées à l'opposé l'une de l'autre, elles tirent les bagues fendues à distance des rétrécissements de section des logements, si bien que ces bagues fendues peuvent se dilater et libérer les tiges.

L'inconvénient majeur de ce mécanisme est son manque de fiabilité dans le temps.

Les caractéristiques élastiques des bouchons risquent en effet d'évoluer dans le temps, ne permettant plus à l'implant de remplir la fonction pour laquelle il a été conçu.

Un effet d'adhérence des bagues fendues sur les tiges est également à craindre, rendant le mécanisme inopérant.

Le document WO 2012/085405 , qui est considéré comme l'état de la technique le plus proche de l'objet de la revendication 1, divulgue:

Un implant à allongement automatique, comportant :
- un corps délimitant intérieurement un logement axial qui s'ouvre d'un côté par une embouchure et qui présente un rétrécissement de section formant sur le corps une face de butée,
- un bouchon annulaire qui est fixé à l'embouchure du logement axial dudit corps et qui présente une face d'arrêt tournée vers ladite face de butée,
- au moins une tige engagée au travers dudit bouchon annulaire et du logement axial dudit corps, et
- une bague fendue qui est montée sur la tige et qui est logée dans le logement axial dudit corps, ladite face de butée présentant une forme biseautée rentrante, et ladite bague fendue présente deux faces d'appui qui sont tournées respectivement vers lesdites faces de butée et d'arrêt et qui présentent des formes telles que ladite bague fendue se comprime sur ladite tige lorsqu'elle s'appuie contre la face de forme biseautée rentrante.

### OBJET DE L'INVENTION

Afin de remédier aux inconvénients précités de l'état de la technique, la présente invention propose un implant à allongement automatique d'architecture simple, fiable et peu encombrant.

Plus particulièrement, on propose selon l'invention un implant tel que défini dans l'introduction, dans lequel lesdites faces de butée et d'arrêt présentent des formes biseautées l'une rentrante et l'autre saillante, et dans lequel ladite bague fendue présente deux faces d'appui qui sont tournées respectivement vers lesdites faces de butée et d'arrêt et qui présentent des formes telles que ladite bague fendue se dilate lorsqu'elle s'appuie contre la face de forme biseautée saillante et qu'elle se comprime sur ladite tige lorsqu'elle s'appuie contre la face de forme biseautée rentrante.

Ainsi, grâce à l'invention, lorsque la tige est poussée dans un sens, elle pousse la bague fendue contre la face rentrante, ce qui comprime la bague fendue sur la tige et bloque donc la tige.

Au contraire, lorsque la tige est tirée dans le sens opposé, elle ramène la bague fendue en appui contre la face saillante. La forme saillante de cette face lui permet de forcer la bague fendue à se dilater, ce qui libère alors la mobilité en translation de la tige.

Aucun risque d'adhérence de la bague fendue sur la tige n'est donc plus à craindre.

Grâce à cette architecture, il n'est par ailleurs plus besoin de ressorts, ce qui assure à l'implant une bonne fiabilité dans la durée.

D'autres caractéristiques avantageuses et non limitatives de l'implant conforme à l'invention sont les suivantes :
- lesdites faces d'appui présentent des formes complémentaires desdites faces de butée et d'arrêt ;
- lesdites faces de butée et d'arrêt présentent des formes coniques ;
- la face interne de ladite bague fendue et une partie au moins de la longueur de ladite tige présentent des crans qui coopèrent ensemble ;
- il est prévu une clavette qui est logée dans un renfoncement latéral du logement axial et dont un méplat s'applique contre un méplat prévu en correspondance sur la tige ;
- ledit bouchon annulaire est incompressible ;
- lorsque l'implant est destiné à une application rachidienne, ledit logement axial est traversant et dans lequel ledit corps délimite un autre logement axial traversant qui est traversé par une autre tige parallèle à ladite tige ;
- il est prévu au moins un capuchon qui est fixé audit corps et qui est enfilé sur une extrémité de ladite tige ;
- lorsque l'implant est destiné à une application sur os long, ledit corps est formé par une seconde tige qui est au moins en partie creuse et qui est située dans le prolongement de ladite tige, ladite tige et ladite seconde tige présentant chacune au moins un trou transversal traversant ; et
- lorsque l'implant est destiné à une application articulaire, ledit corps est formé par une seconde tige qui est au moins en partie creuse et qui est située dans le prolongement de ladite tige, l'une desdites tige et seconde tige présentant alors au moins un trou transversal traversant et l'autre desdites tige et seconde tige portant à son extrémité libre une prothèse articulaire.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique en perspective éclatée d'un premier mode de réalisation d'un implant selon l'invention, destiné à une application sur colonne vertébrale ;
- la figure 2 est une vue schématique en perspective assemblée de l'implant de la figure 1 ;
- la figure 3 est une vue schématique en coupe axiale selon le plan A-A de la figure 2 ;

- la figure 4 est une vue schématique en perspective assemblée de l'implant de la figure 1, sur lequel ont été rapportés deux vis et trois crochets ;
- la figure 5 est une vue schématique en perspective assemblée d'une variante de réalisation de l'implant de la figure 1 ;
- la figure 6 est une vue schématique en perspective d'un second mode de réalisation de l'implant selon l'invention, destiné à une application sur os long ;
- la figure 7 est une vue schématique en coupe de l'implant de la figure 6;
- la figure 8 est une vue schématique en perspective d'un troisième mode de réalisation de l'implant selon l'invention, formant prothèse humérale de l'épaule ;
- la figure 9 est une vue schématique en perspective d'un quatrième mode de réalisation de l'implant selon l'invention, formant prothèse fémorale du genou ;
- la figure 10 est une vue schématique en perspective d'un cinquième mode de réalisation de l'implant selon l'invention, formant prothèse tibiale du genou ;
- la figure 11 est une vue schématique en perspective d'un sixième mode de réalisation de l'implant selon l'invention, formant prothèse fémorale de la hanche.

En préliminaire on notera que les éléments identiques ou similaires des différentes variantes et des différents modes de réalisation de l'invention représentés sur les différentes figures seront, dans la mesure du possible, référencés par les mêmes signes de référence et ne seront pas décrits à chaque fois.

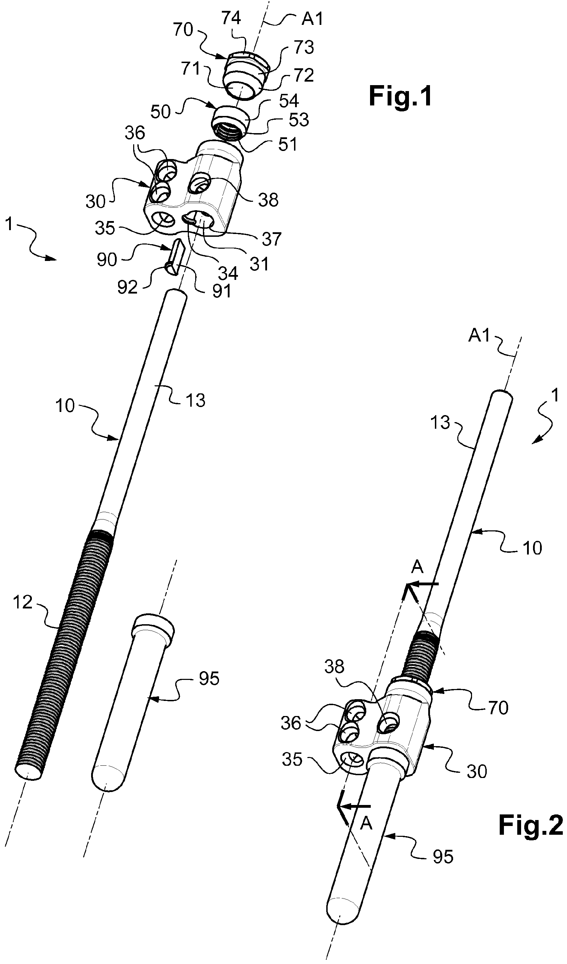

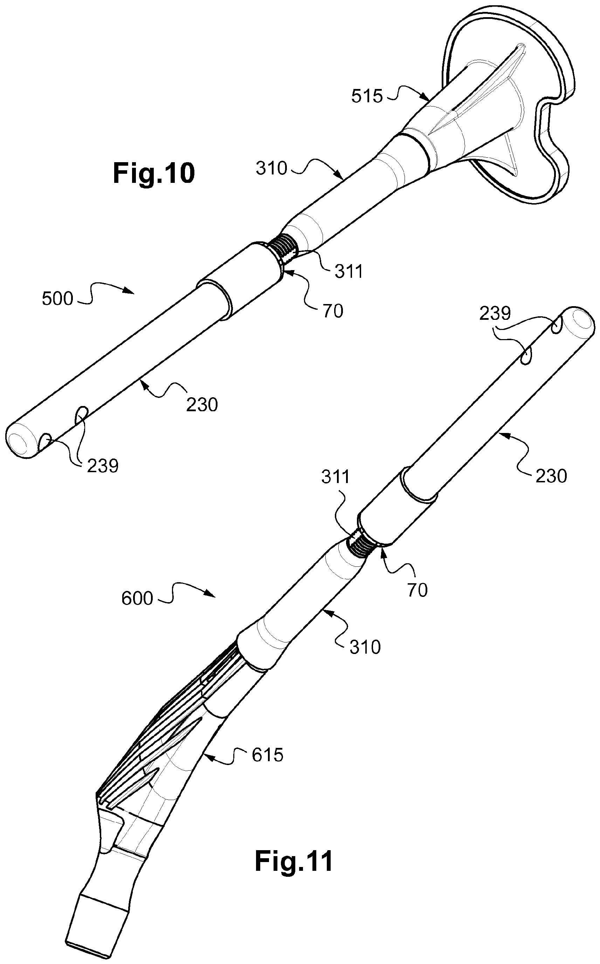

On a représenté sur les figures 1 à 11 six modes de réalisation d'un implant à allongement automatique 1 ; 100 ; 200 ; 300 ; 400 ; 500 ; 600 à fixer dans ou sur un os d'un jeune patient.

Dans ces six modes de réalisation, cet implant est conçu pour accompagner ou forcer la croissance de l'os sur lequel il est rapporté.

Tel qu'il est représenté sur les figures, cet implant à allongement automatique 1 ; 100 ; 200 ; 300 ; 400 ; 500 ; 600 comporte quatre composants principaux, dont une tige mobile 10 ; 110 ; 210 ; 310, un corps 30 ; 130 ; 230, un bouchon annulaire 70, et une bague fendue 50.

La tige mobile 10 ; 110 ; 210 ; 310 est conçue pour être fixée dans ou sur l'os du patient.

Le corps 30 ; 130 ; 230, le bouchon annulaire 70, et la bague fendue 50 sont quant à eux conçus pour permettre à l'implant de s'allonger lorsqu'un effort de traction est exercé sur la tige mobile 10 ; 110 ; 210 ; 310, et pour éviter tout raccourcissement de l'implant lorsqu'un effort de compression est exercé sur la tige mobile 10 ; 110 ; 210 ; 310.

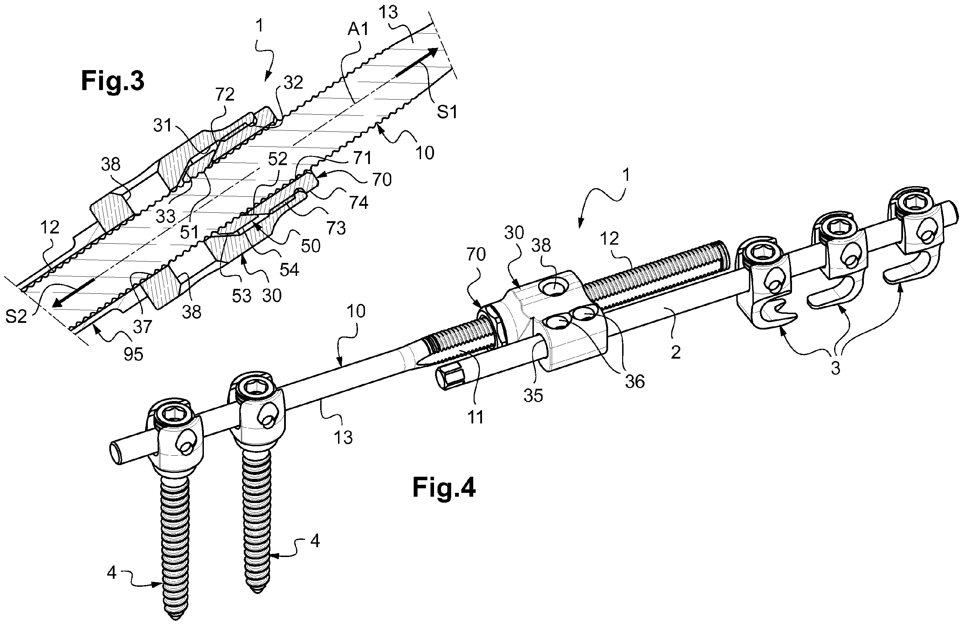

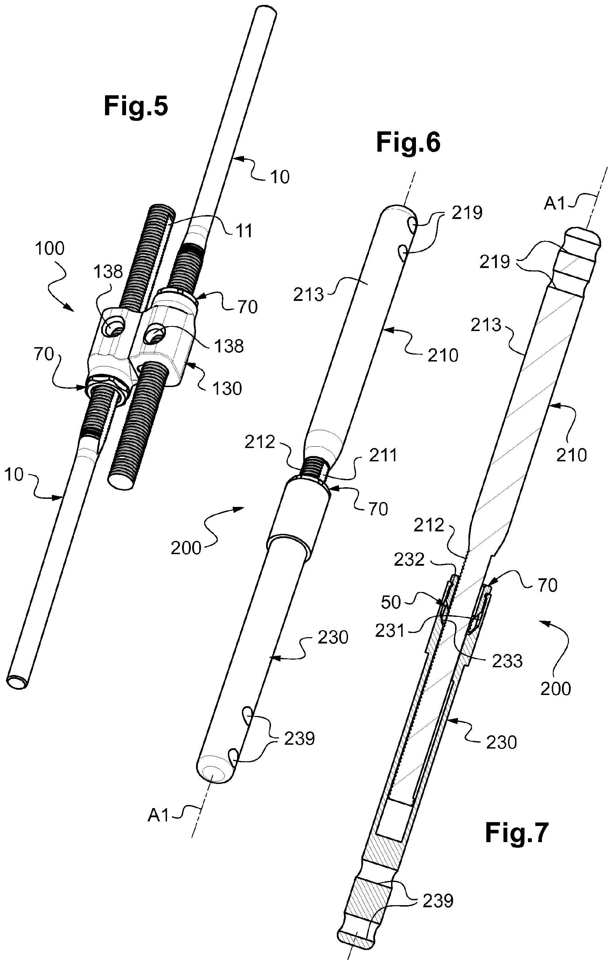

Pour cela, le corps 30 ; 130 ; 230 délimite intérieurement au moins un logement axial 31 ; 231 d'axe A1 qui accueille la bague fendue 50, ce logement axial s'ouvrant d'un côté par une large embouchure 32 ; 232 et présentant un rétrécissement de section formant une face de butée 33 ; 233 (voir figures 3 et 7) pour la bague fendue 50.

Le bouchon annulaire 70 est quant à lui fixé dans l'embouchure 32 ; 232 du logement axial 31 ; 231 du corps 30 ; 130 ; 230 et présente une face d'arrêt 72 tournée vers la face de butée 33 ; 233 précitée (voir figure 3) pour retenir la bague fendue 50 dans le logement axial 31 ; 231.

La tige 10 ; 110 ; 210 ; 310 est enfilée au travers du bouchon annulaire 70, de la bague fendue 50 et du logement axial 31 ; 231 du corps 30 ; 130 ; 230.

Selon une caractéristique particulièrement avantageuse de l'invention, les faces de butée 33 ; 233 et d'arrêt 72 présentent des formes biseautées l'une rentrante et l'autre saillante, et la bague fendue 50 présente deux faces d'appui 52, 53 qui sont tournées respectivement vers ces faces de butée 33 ; 233 et d'arrêt 72.

On définit ici une face de forme saillante, comme une face dont chaque section axiale forme un dièdre d'angle aigu.

On définit par ailleurs une face de forme rentrante, comme une face dont chaque section axiale forme un dièdre d'angle obtus.

Ici, la face de forme saillante est formée par la face d'arrêt 72 du bouchon annulaire 70, tandis que la face de forme rentrante est formée par la face de butée 33 ; 233 du corps 30 ; 130 ; 230.

Ainsi, lorsqu'un effort de traction est appliqué sur la tige 10 ; 110 ; 210 ; 310 (dans le sens S1 sur la figure 3), cette dernière ramène la face d'appui 52 de la bague fendue 50 contre la face d'arrêt 72 du bouchon annulaire 70, ce qui permet de dilater la bague fendue 50 et ainsi donc de libérer la tige.

Au contraire, lorsqu'un effort de compression est appliqué sur la tige 10 ; 110 ; 210 ; 310 (dans le sens S2 sur la figure 3), cette dernière ramène l'autre face d'appui 52 de la bague fendue 50 contre la face de butée 33 ; 233 du corps 30 ; 130; 230, ce qui permet de comprimer la bague fendue 50 sur la tige qui se retrouve alors bloquée.

Ici, et de manière préférentielle, les faces d'appui 52, 53 présentent des formes complémentaires des faces de butée 33 ; 233 et d'arrêt 72.

Avantageusement, les faces d'appui 52, 53 de la bague fendue 50, la face de butée 33 ; 233 du corps 30 ; 130 ; 230 et la face d'arrêt 72 du bouchon annulaire 70 présentent alors des formes tronconiques de révolution autour de l'axe A1. Ces différentes faces tronconiques présentent en outre ici toutes un même angle au sommet, de l'ordre de 60 degrés.

Sur les figures 1 à 4, on a plus particulièrement représenté un premier mode de réalisation de l'implant à allongement automatique 1.

Dans ce mode de réalisation, cet implant constitue un système de connexion 1 permettant à un chirurgien de bloquer au moins une vertèbre d'une colonne vertébrale d'un jeune patient par rapport à une autre vertèbre ou par rapport au bassin de ce patient.

Ce système de connexion 1 est utilisé notamment pour redresser la colonne vertébrale du patient lorsque celle-ci présente une scoliose ou une cyphose prononcée.

Tel qu'il est représenté sur la figure 4, ce système de connexion 1 comporte deux tiges, dont la tige mobile 10 précitée et une tige fixe 2.

Le corps 30 comporte alors, outre le logement axial 31 précité, un conduit 35 d'axe parallèle à l'axe A1 du logement axial 31. Le logement axial 31 et le conduit 35 sont ici tous deux traversants et sont respectivement traversés par la tige mobile 10 et par la tige fixe 2.

L'une des deux tiges, ici la tige fixe 2, est prévue pour être équipée de trois crochets adaptés à être fixés à trois vertèbres cervicales ou dorsales du patient, tandis que l'autre des deux tiges, ici la tige mobile 10, est équipée de deux vis 4 adaptées à être vissées dans les pédicules des vertèbres lombaires du patient.

On parle alors de « montage suspendu » en ce sens que les vertèbres cervicales ou dorsales sont suspendues à l'extrémité libre de la tige fixe 2.

La tige fixe 2 présente ici une forme cylindrique de révolution avec une surface externe lisse.

Elle est réalisée d'une seule pièce en titane.

La tige mobile 10 présente quant à elle une moitié lisse 13 cylindrique de révolution autour de l'axe A1 et de diamètre sensiblement égal à celui de la tige fixe 2, et une moitié crantée 12 symétrique de révolution autour de l'axe A1 et de diamètre supérieur.

Comme le montre la figure 3, les crans sont formés par une succession régulière de convexités et de concavités, qui forment chacune une nervure bombée en saillie ou une gorge incurvée en creux sur la tige mobile 10.

Ici, comme le montre plus particulièrement la figure 4, la moitié crantée 12 de la tige mobile 10 présente un méplat 11 latéral, qui s'étend sur toute sa longueur.

Comme le montre la figure 1, le corps 30 présente quant à lui une forme globale de parallélépipède traversé dans sa plus grande longueur par le logement axial 31 de passage de la tige mobile 10 et par le conduit 35 de passage de la tige fixe 2.

Comme cela apparaît sur la figure 4, le conduit 35 présente un diamètre égal, au jeu de montage près, au diamètre de la tige fixe 2. Il permet ainsi de guider la tige fixe 2 en translation suivant un axe parallèle à l'axe A1.

Le corps 30 présente alors deux alésages taraudés 36 d'axes perpendiculaires à l'axe du conduit 35, qui débouchent d'un côté vers l'extérieur et de l'autre dans le conduit 35.

Ces deux alésages taraudés 36, qui sont ici juxtaposés côte-à-côte, permettent d'accueillir deux vis (non représentées) de telle manière que leurs extrémités puissent s'appuyer contre la tige fixe 2 pour la bloquer dans le conduit 35.

Les embouchures de ces deux alésages taraudés 36 sont ici chanfreinées pour accueillir les têtes de ces vis, de telle manière que ces dernières n'émergent pas à l'extérieur du corps 30.

Comme cela apparaît sur la figure 3, le logement axial 31 débouche quant à lui de part et d'autre du corps 30, d'un côté par la large embouchure 32 et, de l'autre, par un débouché 37 de plus faible diamètre.

Le rétrécissement de section du logement axial 31, qui forme sur le corps 30 ladite face de butée 33, est ici situé à mi-longueur du logement axial 31. Le reste du logement axial 31 présente une section de diamètre constant.

Ainsi, une première partie du logement axial 31, celle située entre le débouché 37 et le rétrécissement de section, présente un diamètre constant égal, au jeu de montage près, au diamètre extérieur de la moitié crantée 12 de la tige mobile 10. De cette manière, cette première partie du logement axial 31 participe au guidage en translation de la tige mobile 10 suivant l'axe A1.

L'autre partie du logement axial 31, celle située entre l'embouchure 32 et le rétrécissement de section, présente quant à elle un diamètre supérieur au diamètre de la tige mobile 10, ce qui lui permet d'accueillir la bague fendue 50 et le bouchon annulaire 70.

Comme le montrent les figures 1 à 4, le corps 30 présente alors deux autres alésages taraudés 38, d'axes perpendiculaires à l'axe A1, qui débouchent chacun d'un côté vers l'extérieur et de l'autre dans la première partie du logement axial 31. Ces deux alésages taraudés 38 sont ici coaxiaux et débouchent donc l'un en face de l'autre à l'intérieur du logement axial 31.

Ces deux alésages taraudés 38 permettent d'accueillir deux vis (non représentées) de telle manière que leurs extrémités puissent s'appuyer de part et d'autre de la tige mobile 10 pour la bloquer dans le logement axial 31.

Les embouchures de ces deux alésages taraudés 38 sont ici aussi chanfreinées pour accueillir les têtes de ces vis, de telle manière que ces dernières n'émergent pas à l'extérieur du corps 30.

Le corps 30 est ici formé d'une seule pièce en titane.

Le bouchon annulaire 70 présente une forme sensiblement de révolution autour de l'axe A1.

Il présente ainsi un conduit central 71 cylindrique de révolution autour de l'axe A1, qui est lisse pour permettre le passage de la tige mobile 10. Ce conduit central 71 présente un diamètre égal, au jeu de montage près, au diamètre extérieur de la moitié crantée 12 de la tige mobile 10. Il participe alors au guidage en translation de cette tige mobile 10 suivant l'axe A1.

Le bouchon annulaire 70 présente une face externe 73 filetée, ce qui lui permet d'être vissé dans l'embouchure 32 taraudée du logement axial 31 du corps 3.

Le bouchon annulaire 70 est alors bordé extérieurement, à une extrémité, par une couronne à 6 pans grâce à laquelle il peut être solidement vissé dans cette embouchure 32, au moyen d'un outil prévu à cet effet (de type clef à pipe).

L'extrémité opposée de ce bouchon annulaire 70, qui constitue ladite face d'arrêt 72, présente une forme biseautée de manière saillante.

Ce bouchon annulaire 70 est ici formé d'une seule pièce en titane, de manière à être indéformable et incompressible.

Telle qu'elle apparaît sur les figures 1 et 3, la bague fendue 50 présente un forme globale de tube d'axe A1 fendu longitudinalement.

Elle présente ainsi une face externe 54 sensiblement cylindrique de révolution autour de l'axe A1. Elle présente par ailleurs une face interne 51 sensiblement de révolution autour de l'axe A1, qui est ici crantée de manière à pouvoir engrener les crans de la tige mobile 10. Elle présente enfin deux faces d'extrémité, l'une saillante et l'autre rentrante, qui forment lesdites faces d'appui 52,53.

La fente prévue permet à la bague fendue 50 de se comprimer radialement ou de se dilater radialement autour de l'axe A1. Cette fente présente une largeur telle que, lorsque la bague fendue 50 est comprimée et que les deux bords de cette fente se rejoignent, la face intérieure 51 de la bague fendue 50 présente un diamètre moyen inférieur au diamètre moyen de la moitié crantée 12 de la tige mobile 10.

Cette bague fendue 50 est ici formée d'une seule pièce en titane.

De manière avantageuse, comme le montre la figure 1, le système de connexion 1 comporte en outre une clavette 90 et un capuchon 95.

La clavette 90 est prévue pour bloquer toute rotation de la tige mobile 10 par rapport au corps 30 autour de l'axe A1.

Elle présente une forme de demi-cylindre, si bien qu'elle comporte une face plane qui définit un méplat 91.

Elle est adaptée à être logée dans un renfoncement latéral 34 de forme complémentaire prévu en creux dans le logement axial 31, du côté du débouché 37 de ce logement axial 31.

Ainsi, une fois la clavette 90 logée dans le renfoncement latéral 34, la tige mobile 10 ne peut être engagée dans le logement axial 31 que de telle manière que son méplat 11 s'appuie contre le méplat 91 de la clavette 90, ce qui bloque ensuite toute rotation de la tige mobile 10 autour de l'axe A1.

Pour retenir la clavette 90 dans le renfoncement latéral 34, une des extrémités de cette clavette 90 est bordée, sur sa face opposée au méplat 91, par une demi-couronne 92 qui se loge dans une gorge prévue en correspondance dans le renfoncement latéral 34.

Comme le montrent les figures 1 à 3, le capuchon 95 présente quant à lui une forme de tube ouvert à une extrémité et fermé à l'extrémité opposée par une paroi hémisphérique.

Il est fixé par son extrémité ouverte autour du débouché 37 du logement axial 31 (par exemple par soudage ou collage), de manière à protéger la moitié crantée 12 de la tige mobile 10. Ainsi, aucune chair ne vient se déposer entre les crans de la tige mobile 10, ce qui permet une bonne coopération de ces crans avec ceux de la bague fendue 50.

Le capuchon 95 présente un diamètre intérieur égal, au jeu de montage près, au diamètre extérieur de la moitié crantée 12 de la tige mobile 10, si bien qu'il participe au guidage en translation de cette tige mobile 10 suivant l'axe A1.

Il est ici formé d'une seule pièce en titane.

Le système de connexion 1 est livré de manière à présenter une longueur réduite, c'est-à-dire de telle manière que sa moitié crantée 12 débouche à peine de l'embouchure 32 du logement axial 31 du corps 30. Il présente ainsi une grande réserve d'allongement.

Il est livré avec deux vis vissées dans les alésages taraudés 38 pour bloquer la tige mobile 10 dans le corps 30.

La mise en place de ce système de connexion 1 sur les vis 4 et crochets 3 (qui ont préalablement été fixés aux pédicules des vertèbres lombaires et aux vertèbres cervicales ou dorsales du patient) est réalisée sans retirer ces vis de manière que la tige mobile 10 reste bloquée en position.

Cette mise en place nécessite cependant d'ajuster la forme et la longueur du système de connexion 1.

La forme est alors ajustée en cintrant au besoin la tige fixe 2 et la partie lisse 13 de la tige mobile 10, ce qui assure une courbure régulière de l'ensemble du système de connexion 1.

La longueur est quant à elle ajustée en coupant la tige fixe 2 à la taille souhaitée, sans utiliser le potentiel d'allongement de la tige mobile 10, ce qui permet à l'ensemble de la moitié crantée 12 de la tige mobile 10 de servir de réserve de croissance.

A l'issue de cette opération de mise en place du système de connexion 1 sur les vis 4 et crochets 3, les vis vissées dans les alésages taraudés 38 du corps 30 peuvent soit être laissées en place, soit être retirées par le chirurgien.

Lorsqu'elles sont laissées en place, ces vis bloquent tout allongement automatique du système de connexion 1. Des opérations ultérieures seront donc nécessaires pour l'allonger en dévissant puis revissant ces vis. Ce système de connexion 1 ne sera pas pour autant inutile puisque, au cours de chacune de ces opérations ultérieures, la bague fendue 50 évitera que le système de connexion 1 ne puisse se rétrécir.

En revanche, lorsque ces vis sont retirées par le chirurgien, le système de connexion 1 pourra suivre automatiquement la croissance de la colonne vertébrale du patient, en s'allongeant au fur et à mesure de cette croissance. Cet allongement pourra éventuellement être provoqué (sans opération chirurgicale), en demandant au patient de réaliser des mouvements particuliers d'étirement de son tronc, avec l'aide éventuelle d'une tierce personne.

Sur la figure 5, on a représenté une variante de réalisation du système de connexion 100 représenté sur les figures 1 à 4.

Dans cette variante, le système de connexion 100 comporte, non pas une, mais deux tiges mobiles 10 identiques à celle représentée sur la figure 1.

Son corps 130 délimite alors, non pas un, mais deux logements identiques et parallèles, dont les embouchures sont orientées à l'opposé.

Ces logements accueillent alors chacun une bague fendue, un bouchon annulaire 70, et une clavette identiques à ceux représentés sur la figure 1.

Le corps 130 comporte par ailleurs deux paires d'alésages taraudés 138 adaptés à accueillir des vis qui permettent de verrouiller les deux tiges mobiles 10 en position fixe.

Dans cette variante, le potentiel d'allongement du système de connexion 100 est deux fois supérieur à celui du système de connexion 1 représenté sur les figures 1 à 4.

Sur les figures 6 et 7, on a représenté un second mode de réalisation de l'implant à allongement automatique 200 selon l'invention.

Dans ce mode de réalisation, cet implant constitue un axe de liaison 200 permettant à un chirurgien de connecter deux parties d'un os long.

Par os long, on entend tout type d'os de longueur très largement supérieure à son diamètre moyen (par exemple le tibia, l'humérus, les os métacarpiens, ...).

Dans ce mode de réalisation, la tige mobile 210 présente une moitié crantée 212 identique à la moitié crantée 12 de la tige mobile 10 représentée sur la figure 1. Sa moitié lisse 213 présente en revanche un diamètre différent (ici supérieur au diamètre de sa partie crantée 212), ajusté au diamètre du canal médullaire de l'os dans lequel il est prévu de l'engager.

Le corps 230 de cet axe de liaison 200 présente ici une forme de tige de diamètre identique à celui de la moitié lisse 213 de la tige mobile 210.

Le logement axial 231 prévu à l'intérieur de ce corps 230 pour accueillir la moitié crantée 212 de la tige mobile 210 débouche ici uniquement sur une extrémité du corps 230. Il présente, du côté de son embouchure 232, une forme identique à celle du logement axial 31 du corps 30 représenté sur la figure 1. Il se prolonge en revanche à l'intérieur du corps 230, si bien que le corps 230 présente une fonction de capuchon protecteur.

Ce logement axial 231 accueille une bague fendue 50, un bouchon annulaire 70, et une clavette identiques à ceux représentés sur la figure 1.

Pour leur fixation à l'os, la tige mobile 210 et le corps 230 présentent chacun deux trous traversants 219, 239 d'axes perpendiculaires à l'axe A1 de la tige mobile 210. Ces trous traversants 219, 239 permettent ainsi de fixer la tige mobile 210 et le corps 230 aux deux parties de l'os, au moyen de vis.

Ainsi, l'axe de liaison 200 peut alors être utilisé sur un os long en vue de l'allonger.

Pour cela, l'os est découpé en deux parties. La tige mobile 210 est alors engagée dans le canal médullaire de l'une de ces parties, tandis que le corps 230 est engagé dans le canal médullaire de l'autre partie de l'os.

Ils y sont ensuite fixés par des clous.

L'allongement de l'os est ensuite réalisé de manière progressive, d'environ 1 millimètre par jour, de telle sorte que les deux parties de l'os puissent se joindre mais qu'elles n'aient pas le temps de se solidifier (on profite donc de la période de consolidation de l'os pour l'allonger).

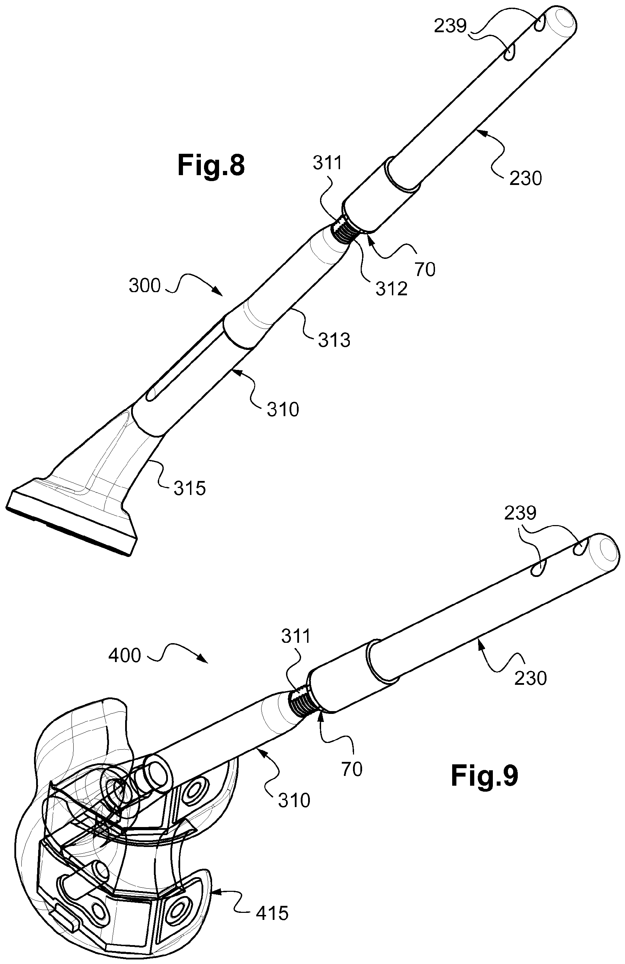

Sur les figures 8 à 11, on a représenté d'autres mode de réalisation de l'invention, dans lesquels l'implant à allongement automatique présente une fonction de prothèse articulaire.

Ainsi, sur la figure 8, on a représenté un troisième mode de réalisation de l'implant à allongement automatique 300 selon l'invention.

Dans ce mode de réalisation, cet implant présente une fonction de prothèse humérale de l'épaule.

Son corps présente une forme identique au corps 230 représenté sur la figure 6. Il est ainsi adapté à être fixé dans le canal médullaire de l'humérus du patient et à accueillir une bague fendue 50, un bouchon annulaire 70, et une clavette identiques à ceux représentés sur la figure 1.

Sa tige 310 présente en revanche une forme différente de celles décrites précédemment.

Dans ce mode de réalisation, la tige mobile 310 présente ainsi une moitié crantée 312 identique à la moitié cranté 12 de la tige mobile 10 représentée sur la figure 1. Sa moitié lisse 313 présente en revanche un diamètre qui s'accroit vers son extrémité libre.

Elle présente en outre, en creux dans son extrémité libre, un logement conique qui permet d'y fixer une prothèse d'épaule 315.

Sur les figures 9 à 11, on a respectivement représenté un quatrième, un cinquième et un sixième mode de réalisation de l'implant à allongement automatique 400 ; 500 ; 600 selon l'invention.

Dans ces modes de réalisation, cet implant présente respectivement des fonctions de prothèse fémorale du genou, de prothèse tibiale du genou et de prothèse fémorale de la hanche.

Dans ces trois modes, le corps 230, la tige mobile 310, la bague fendue 50, le bouchon annulaire 70, et la clavette sont identiques à ceux de l'implant représenté sur la figure 8.

Seule la prothèse articulaire fixée dans le logement conique de la tige mobile 310 diffère. Comme cela apparaît respectivement sur les figures 9 à 11, il s'agit alors d'une prothèse fémorale de genoux 415, d'une prothèse tibiale de genou 515 ou d'une prothèse fémorale de hanche 615.

La présente invention n'est nullement limitée aux modes de réalisation décrits et représentés, mais l'homme du métier saura y apporter toute variante conforme à son esprit.

On pourrait en particulier prévoir que l'un et/ou l'autre des composants de l'implant à allongement automatique soit réalisé en matière plastique (notamment en peek, c'est-à-dire en polyétheréthercétone) ou en matériau composite (par exemple à base de fibres de carbone noyées dans une base en peek).

Selon une autre variante, on aurait pu prévoir que le bouchon annulaire se visse, non pas à l'intérieur de l'embouchure du corps, mais à l'extérieur de celui-ci, en le recouvrant. On aurait pu également prévoir de l'y fixer autrement, par exemple par collage ou soudage

Selon une autre variante, on aurait pu prévoir que les faces de butée, d'arrêt et d'appui présentent des formes autres que conique de révolution, par exemple des formes pyramidales. On aurait aussi pu prévoir que la bague fendue présente une forme annulaire de section circulaire.

Encore en variante, on aurait pu prévoir que la tige mobile et la bague fendue ne soient pas crantées, mais soient complètement lisses, auquel cas le blocage de la tige mobile serait toutefois moins fiable.

On aurait aussi pu prévoir que les crans présentent non pas des sections en forme de vagues régulières, mais des sections par exemple en forme de triangles (ce qui faciliterait le passage de la bague fendue d'un cran à l'autre dans un sens, mais bloquerait ce passage dans l'autre sens).

## Revendications

1. Implant à allongement automatique (1 ; 100 ; 200 ; 300 ; 400 ; 500 ; 600), comportant :
- un corps (30 ; 130 ; 230) délimitant intérieurement un logement axial (31 ; 231) qui s'ouvre d'un côté par une embouchure (32 ; 232) et qui présente un rétrécissement de section formant sur le corps (30 ; 130 ; 230) une face de butée (33 ; 233),
- un bouchon annulaire (70) qui est fixé à l'embouchure (32 ; 232) du logement axial (31 ; 231) dudit corps (30 ; 130 ; 230) et qui présente une face d'arrêt (72) tournée vers ladite face de butée (33 ; 233),
- au moins une tige (10; 110; 210; 310) engagée au travers dudit bouchon annulaire (70) et du logement axial (31 ; 231) dudit corps (30 ; 130 ; 230), et
- une bague fendue (50) qui est montée sur la tige (10 ; 110 ; 210 ; 310) et qui est logée dans le logement axial (31 ; 231) dudit corps (30 ; 130 ; 230),
**caractérisé en ce que** lesdites faces de butée (33 ; 233) et d'arrêt (72) présentent des formes biseautées l'une rentrante et l'autre saillante, et **en ce que** ladite bague fendue (50) présente deux faces d'appui (52, 53) qui sont tournées respectivement vers lesdites faces de butée (33 ; 233) et d'arrêt (72) et qui présentent des formes telles que ladite bague fendue (50) se dilate lorsqu'elle s'appuie contre la face de forme biseautée saillante et qu'elle se comprime sur ladite tige (10; 110; 210; 310) lorsqu'elle s'appuie contre la face de forme biseautée rentrante.

2. Implant à allongement automatique (1 ; 100 ; 200 ; 300 ; 400 ; 500 ; 600) selon la revendication précédente, dans lequel lesdites faces d'appui (52, 53) présentent des formes complémentaires desdites faces de butée (33 ; 233) et d'arrêt (72).

3. Implant à allongement automatique (1 ; 100 ; 200 ; 300 ; 400 ; 500 ; 600) selon l'une des revendications précédentes, dans lequel lesdites faces de butée (33 ; 233) et d'arrêt (72) présentent des formes coniques.

4. Implant à allongement automatique (1 ; 100 ; 200 ; 300 ; 400 ; 500 ; 600) selon l'une des revendications précédentes, dans lequel la face interne (51) de ladite bague fendue (50) et une partie au moins de la longueur de ladite tige (10 ; 110 ; 210 ; 310) présentent des crans qui coopèrent ensemble.

5. Implant à allongement automatique (1 ; 100 ; 200 ; 300 ; 400 ; 500 ; 600) selon l'une des revendications précédentes, dans lequel il est prévu une clavette (90) qui est logée dans un renfoncement latéral (34) du logement axial (31) et dont un méplat (91) s'applique contre un méplat (11 ; 211 ; 311) prévu en correspondance sur la tige (10 ; 110 ; 210 ; 310).

6. Implant à allongement automatique (1 ; 100 ; 200 ; 300 ; 400 ; 500 ; 600) selon l'une des revendications précédentes, dans lequel ledit bouchon annulaire (70) est incompressible.

7. Implant à allongement automatique (1 ; 100) selon l'une des revendications 1 à 6 destiné à une application rachidienne, dans lequel ledit logement axial (31) est traversant et dans lequel ledit corps (30) délimite un autre logement axial (35) traversant qui est traversé par une autre tige (2) parallèle à ladite tige (10 ; 110).

8. Implant à allongement automatique (1) selon la revendication précédente, dans lequel il est prévu au moins un capuchon (95) qui est fixé audit corps (30) et qui est enfilé sur une extrémité (12) de ladite tige (10).

9. Implant à allongement automatique (200) selon l'une des revendications 1 à 6 destiné à une application sur os long, dans lequel ledit corps est formé par une seconde tige (230) qui est au moins en partie creuse et qui est située dans le prolongement de ladite tige (210), ladite tige (210) et ladite seconde tige (230) présentant chacune au moins un trou transversal traversant (219, 239).

10. Implant à allongement automatique (300 ; 400 ; 500 ; 600) selon l'une des revendications 1 à 6 destiné à une application articulaire, dans lequel ledit corps est formé par une seconde tige (230) qui est au moins en partie creuse et qui est située dans le prolongement de ladite tige (210) et dans lequel l'une desdites tige (210) et seconde tige (230) présente au moins un trou transversal traversant (219, 239) et l'autre desdites tige (210) et seconde tige (230) porte à son extrémité libre une prothèse articulaire (315 ; 415 ; 515 ; 615).

## Patentansprüche

1. Selbstexpandierendes Implantat (1; 100; 200; 300; 400; 500; 600) mit
- einem Körper (30; 130; 230), in dessen Inneren ein axialer Raum (31; 231) abgegrenzt ist, der nach einer Seite hin durch eine Austrittsöffnung (32; 232) offen ist und der eine Verjüngung aufweist, die auf dem Körper (30; 130; 230) eine Anschlagsfläche (33; 233) ausbildet,
- einem ringförmigen Stopfen (70), der an der Austrittsöffnung (32; 232) des axialen Raums (31; 231) des Körpers (30; 130; 230) angebracht ist und der eine zur Anschlagsfläche (33; 233) zeigende Sperrfläche (72) aufweist,
- wenigstens einer durch den ringförmigen Stopfen (70) und den axialen Raum (31; 231) des Körpers (30; 130; 230) hindurchgeführten Stange (10; 110; 210; 310) und
- einem geschlitzten Ring (50), der auf der Stange (10; 110; 210; 310) angebracht und im axialen Raum (31; 231) des Körpers (30; 130; 230) untergebracht ist,
**dadurch gekennzeichnet, daß** die Anschlagsfläche (33; 233) und die Sperrfläche (72) abgeschrägte Formen, eine nach innen führende bzw. eine nach außen führende, aufweisen und daß der geschlitzte Ring (50) zwei Andruckflächen (52, 53) aufweist, die zur Anschlagsfläche (33; 233) bzw. zur Sperrfläche (72) zeigen und die so geformt sind, daß sich der geschlitzte Ring (50) aufweitet, wenn er gegen die Fläche mit nach außen führender abgeschrägter Form gedrückt wird, und daß er sich auf der Stange (10; 110; 210; 310) zusammenzieht, wenn er gegen die Fläche mit nach innen führender abgeschrägter Form gedrückt wird.

2. Selbstexpandierendes Implantat (1; 100; 200; 300; 400; 500; 600) gemäß dem vorangehenden Anspruch, **dadurch gekennzeichnet, daß** die beiden Andruckflächen (52, 53) zur Anschlagsfläche (33; 233) bzw. zur Sperrfläche (72) komplementäre Formen aufweisen.

3. Selbstexpandierendes Implantat (1; 100; 200; 300; 400; 500; 600) gemäß einem der vorangehenden Ansprüche, **dadurch gekennzeichnet, daß** die Anschlagsfläche (33; 233) und die Sperrfläche (72) konische Formen aufweisen.

4. Selbstexpandierendes Implantat (1; 100; 200; 300; 400; 500; 600) gemäß einem der vorangehenden Ansprüche, bei dem die innere Seite (51) des geschlitzten Rings (50) und wenigstens ein Teil der Länge der Stange (10; 110; 210; 310) miteinander zusammenwirkende Einkerbungen aufweisen.

5. Selbstexpandierendes Implantat (1; 100; 200; 300; 400; 500; 600) gemäß einem der vorangehenden Ansprüche, **dadurch gekennzeichnet, daß** eine Paßfeder (90) vorgesehen ist, die in einer seitlichen Verstärkung (34) des axialen Raums (31) untergebracht ist und deren eine Abflachung (91) gegen eine an der Stange (10; 110; 210; 310) entsprechend vorgesehene Abflachung (11; 211; 311) drückt.

6. Selbstexpandierendes Implantat (1; 100; 200; 300; 400; 500; 600) gemäß einem der vorangehenden Ansprüche, **dadurch gekennzeichnet, daß** der ringförmige Stopfen (70) nicht komprimierbar ist.

7. Selbstexpandierendes Implantat (1; 100) gemäß einem der Ansprüche 1 bis 6, das für eine spinale Anwendung vorgesehen ist, bei dem der axiale Raum (31) einen Durchgang bildet und bei dem im Körper (30) ein weiterer axialer Raum (35) abgegrenzt ist, durch den eine zur besagten Stange (10; 110) parallele weitere Stange (2) geführt ist.

8. Selbstexpandierendes Implantat (1) gemäß dem vorangehenden Anspruch, bei dem wenigstens eine Kappe (95) vorgesehen ist, die am besagen Körper (30) befestigt ist und die auf ein Ende (12) der besagten Stange (10) gestülpt ist.

9. Selbstexpandierendes Implantat (200) gemäß einem der Ansprüche 1 bis 6 für eine Anwendung bei einem langen Knochen, bei dem der Körper durch eine zweite Stange (230) gebildet ist, die wenigstens teilweise hohl ist und die in Verlängerung der besagten Stange (210) angeordnet ist, wobei die Stange (210) und die zweite Stange (230) jeweils wenigstens ein querliegendes Durchgangsloch (219, 239) aufweisen.

10. Selbstexpandierendes Implantat (300; 400; 500; 600) gemäß einem der Ansprüche 1 bis 6 für eine Gelenkanwendung, bei dem der Körper durch eine zweite Stange (230) gebildet ist, die wenigstens teilweise hohl ist und die in Verlängerung der besagten Stange (210) angeordnet ist, wobei die eine der beiden Stangen, die Stange (210) oder die zweite Stange (230), wenigstens ein querliegendes Durchgangsloch (219, 239) aufweist und die andere der beiden Stangen (230 bzw. 210) an deren freiem Ende eine Gelenkprothese (315; 415; 515; 615) trägt.

## Claims

1. An automatically lengthening implant (1; 100; 200; 300; 400; 500; 600) comprising:
• a body (30; 130; 230) defining internally an axial housing (31; 231) that opens out, at one end, via a mouth (32; 232), and that presents a narrowing of section so as to form an abutment face (33; 233) on the body (30; 130; 230) ;
• an annular plug (70) that is fastened to the mouth (32; 232) of the axial housing (31; 231) of said body (30; 130; 230) and that presents a stop face (72) facing towards said abutment face (33; 233);
• at least one rod (10; 110; 210; 310) that is engaged through said annular plug (70) and the axial housing (31; 231) of said body (30; 130; 230); and
• a split ring (50) that is mounted on the rod (10; 110; 210; 310) and that is housed in the axial housing (31; 231) of said body (30; 130; 230);
the implant being **characterized in that** said abutment face (33; 233) and stop face (72) present shapes that are chamfered, one reentrant and the other projecting, and **in that** said split ring (50) presents two thrust faces (52, 53) that face respectively towards said abutment face (33; 233) and towards said stop face (72) and that present shapes such that said split ring (50) expands when it is pressed against the chamfered face of projecting shape and compresses against said rod (10; 110; 210; 310) when it is pressed against the chamfered face of reentrant shape.

2. An automatically lengthening implant (1; 100; 200; 300; 400; 500; 600) according to the preceding claim, wherein said thrust faces (52, 53) present shapes that are complementary to said abutment face (33; 233) and said stop face (72).

3. An automatically lengthening implant (1; 100; 200; 300; 400; 500; 600) according to either preceding claim, wherein said abutment face (33; 233) and said stop face (72) present conical shapes.

4. An automatically lengthening implant (1; 100; 200; 300; 400; 500; 600) according to any preceding claim, wherein the inside face (51) of said split ring (50) and at least a portion of the length of said rod (10; 110; 210; 310) present notches that co-operate together.

5. An automatically lengthening implant (1; 100; 200; 300; 400; 500; 600) according to any preceding claim, wherein a pin (90) is received in a lateral setback (34) of the axial housing (31) and has a flat (91) pressing against a flat (11; 211; 311) provided correspondingly on the rod (10; 110; 210; 310).

6. An automatically lengthening implant (1; 100; 200; 300; 400; 500; 600) according to any preceding claim, wherein said annular plug (70) is incompressible.

7. An automatically lengthening implant (1; 100) according to any one of claims 1 to 6 for application to a spine, wherein said axial housing (31) is a through housing and wherein said body (30) defines another through axial housing (35) passing another rod (2) parallel to said rod (10; 110).

8. An automatically lengthening implant (1) according to the preceding claim, wherein at least one cap (95) is fastened to said body (30) and is engaged on a second end (12) of said rod (10).

9. An automatically lengthening implant (200) according to one of claims 1 to 6, for application to a long bone, wherein said body is formed by a second rod (230) that is hollow at least in part and that is situated to extend said rod (210), said rod (210) and said second rod (230) each presenting at least one transverse through hole (219, 239).

10. An automatically lengthening implant (300; 400; 500; 600) according to any one of claims 1 to 6 for application to a joint, wherein said body is formed by a second rod (230) that is hollow, at least in part, and that is situated extending said rod (210), and wherein one of said rod (210) and said second rod (230) presents at least one transverse through hole (219, 239) and the other one of said rod (210) and of said second rod (230) carries a joint prosthesis (315; 415; 515; 615) at its free end.
